# EUROPÄISCHE PATENTSCHRIFT

(11) **EP 2 895 131 B1**
(45) Veröffentlichungstag und Bekanntmachung des Hinweises auf die Patenterteilung: **31.08.2016**
(21) Anmeldenummer: 13753888.0
(22) Anmeldetag: 02.09.2013
(51) Int. Cl.: A61G 11/00, A61G 7/05

(54) **WÄRMETHERAPIEGERÄT**
HEAT THERAPY DEVICE
APPAREIL DE THERMOTHÉRAPIE

(30) Priorität: 14.09.2012 DE 102012216473
(43) Veröffentlichungstag der Anmeldung: 22.07.2015
(73) Patentinhaber: Drägerwerk AG & Co. KGaA, 23558 Lübeck (DE)
(72) Erfinder: KOCH, Jochim, 23909 Ratzeburg (DE); HAMPE, Markus, 23562 Lübeck (DE); KOHNKE, Claudia, 23562 Lübeck (DE)
(74) Vertreter: Kettenbeil, Roxane Henriette
(86) Internationale Anmeldenummer: PCT/EP2013/068116
(87) Internationale Veröffentlichungsnummer: WO 2014/040876

(56) Entgegenhaltungen:
- EP-A2- 2 514 399
- WO-A1-99/12511
- US-B1- 6 234 954

## Beschreibung

Die vorliegende Erfindung betrifft ein Wärmetherapiegerät, insbesondere Inkubator, Wärmebettchen oder Offene Pflegeeinheit, zur Behandlung von Neugeborenen mit einer von Seitenwänden umrandeten Liegefläche zur Aufnahme eines Neugeborenen, wobei wenigstens ein Seitenwandteil mit wenigstens einem zugehörigen Scharnier nach unten schwenkbar aufgehängt ist und ein lösbarer Verriegelungsmechanismus zum Verriegeln des Seitenwandteils in der aufrechten geschlossenen Stellung vorhanden ist, der einen Schließvorsprung, eine komplementär geformte Aufnahme, die an einer Seite offen ist, und eine Führungskurve aufweist, die dazu ausgestaltet sind, so dass der Schließvorsprung bei Erreichen der geschlossenen Stellung des Seitenwandteils durch die offene Seite zum formschlüssigen Eingriff in die Aufnahme eintreten kann, um so das Seitenwandteil in der aufrechten geschlossenen Stellung an einer feststehenden Struktur des Wärmetherapiegeräts zu verriegeln.

Bei vielen Wärmetherapiegeräten können die Seitenwände für einen besseren Zugang zum Säugling geöffnet werden. Wärmetherapiegeräte mit aufklappbaren Seitenwänden sind zum Beispiel in U.S. 6,231,499 B1 und WO 2009/073693 A1 beschrieben. In der geschlossenen Position der Seitenwände müssen diese sicher arretiert gehalten werden, um ein Herausfallen des Patienten zu verhindern. Dazu gibt es in der Norm IEC 60601-2-19 und -2-21 entsprechende Anforderungen und Tests. In den zuvor erwähnten beiden Dokumenten sind die Verschlussmechanismen zum Halten der Seitenwände in der geschlossenen Position nicht näher beschrieben.

· Aus US 6 234 954 B1 ist ein Wärmetherapiegerät mit den Merkmalen des Oberbegriffs von Patentanspruch 1 bekannt.

Bei vielen heute in Benutzung befindlichen Typen von Inkubatoren mit aufklappbaren Seitenwandteilen werden zum Arretieren des Seitenwandteils, in der geschlossenen Stellung einfache. Rastverschlüsse verwendet, die entweder an der Oberkante der daneben befindlichen Seitenwand oder an einer Seitenkante des Kompartments (Aufenthaltsräumlichkeit des Patienten, beispielsweise umgrenzt von einer Inkubatorhaube) einrasten. Dort werden sie durch einen Verschluss verriegelt. Zur vollständigen Arretierung ist auf jeder Seite des Seitenwandteils je ein Verschluss vorhanden. Damit sind zum Ver- und Entriegeln aber zwei Hände oder mindestes zwei aufeinanderfolgende Betätigungsschritte mit einer Hand erforderlich. Im Hinblick auf die Handhabung wäre es aber wünschenswert, das Öffnen durch einen einzigen Betätigungsschritt mit einer Hand bewirken zu können. Außerdem erfordert dieser Verschlussmechanismus das Vorhandensein einer festen Inkubatorhaube oder Kompartments, mit der das Seitenwandteil verriegelt werden kann.

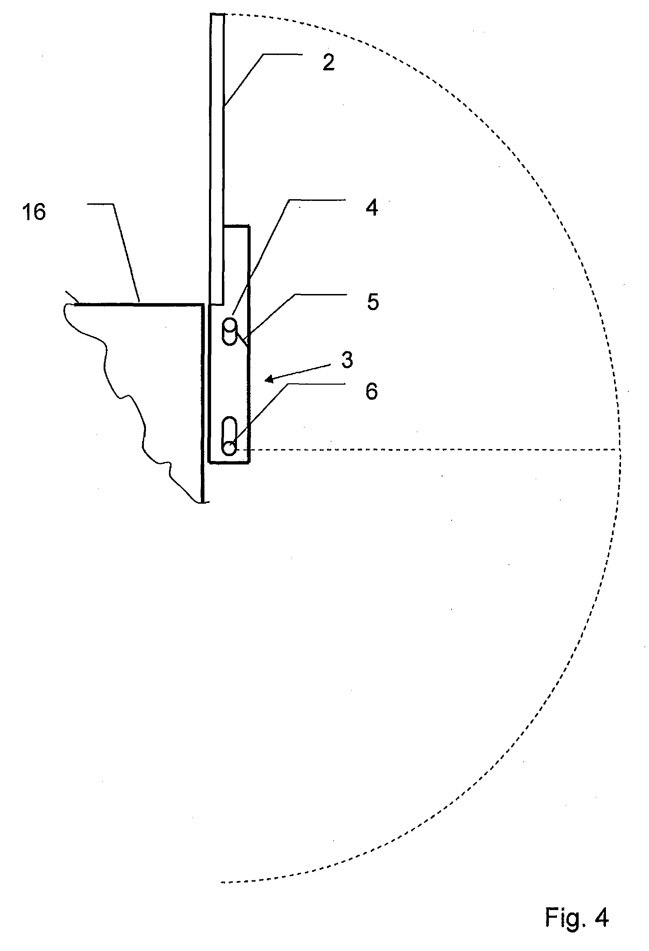

In der offenen Pflege, bei der normalerweise keine den Patientenraum abdeckende Haube verwendet wird, hat es sich durchgesetzt, die Seitenwandteile durch einen Verschlussmechanismus zu verriegeln, der in die Scharniere integriert ist, mit denen die Seitenwandteile nach unten schwenkbar aufgehängt sind. Der Aufbau eines solchen verriegelbaren Scharniers ist in Fig. 4 schematisch dargestellt. Gezeigt ist ein Rand der Liegefläche 16 eines Wärmetherapiegerätes, an dem seitlich ein Seitenwandteil 2 durch ein Scharnier 3 schwenkbar aufgehängt ist. Das Scharnier 3 ist um eine mit dem Wärmetherapiegerät verbundene Schwenkachse 6 schwenkbar. Die Schwenkachse 6 ist in einer Aufnahme des Scharniers geführt, die im Querschnitt die Form eines Langlochs hat. Daher kann das Scharnier bei aufrecht stehendem Seitenwandteil 2 mit diesen angehoben bzw. abgesenkt werden, wobei sich die Schwenkachse 6 in der Langlochaufnahme verschiebt. Das Scharnier ist ferner mit einem Zapfen 4 versehen, der in eine mit dem Wärmetherapiegerät fest verbundene Nut eingreifen kann. Durch Anheben des Seitenwandteils 2 wird das Scharnier soweit angehoben, dass der Zapfen 4 nach oben aus der Nut freikommt, wonach das Seitenwandteil 2 frei schwenkbar ist. Zum Schließen des Seitenwandteils wird dieses zurückgeschwenkt. Kurz vor Erreichen der geschlossenen Stellung kommt der Zapfen 4 mit einer Führungskurve 5 in Berührung, die dafür sorgt, dass das Scharnier mit dem Seitenwandteil 2 daran durch Gleiten des Zapfens 4 auf der Führungskurve 5 soweit angehoben wird, dass der Zapfen 4 in eine Lage oberhalb der Nut kommt, wonach sich das Seitenwandteil 2 und das Scharnier 3 soweit absenken, bis der Zapfen 4 in der Nut aufliegt und damit mit dieser in Eingriff steht, wodurch das Scharnier 3 blockiert ist. Der Oberbegriff von Patentanspruch 1 basiert auf einem Wärmetherapiegerät mit derart verriegelbaren Seitenwandteilen.

Diese Art des Verriegelns des Seitenwandteils in der geschlossenen Stellung ist jedoch von der Handhabung her nicht optimal und erzeugt ein mechanisches Rucken und Schließgeräusche, wenn der Zapfen über die Kante der Führungskurve gleitet, wonach das ganze Seitenwandteil und das Scharnier abwärts fällt, bis der Zapfen auf den Boden der Nut auftrifft.

Es ist Aufgabe der vorliegenden Erfindung, ein Wärmetherapiegerät mit wegklappbaren Seitenwandteilen so auszugestalten, dass das Arretieren der Seitenwandteile in der geschlossenen Stellung in Bezug auf die Handhabung sehr einfach auszuführen ist und möglichst wenig Erschütterungen und Geräusche verursacht.

Zur Lösung dieser Aufgabe dient das Wärmetherapiegerät mit den Merkmalen des Patentanspruchs 1. Vorteilhafte Ausführungsformen sind in den Unteransprüchen aufgeführt.

Erfindungsgemäß ist vorgesehen, dass der Schließvorsprung oder die Aufnahme geradlinig beweglich in Richtung ihrer Verbindungsgeraden zur Schwenkachse des Scharniers aufgehängt ist, so dass der Schließvorsprung in eine Stellung vor der offenen Seite der Aufnahme beweglich ist, und mit einer Vorspannungseinrichtung versehen ist, die dafür sorgt, dass, wenn Schließvorsprung und offene Seite der Aufnahme zueinander ausgerichtet sind, Schließvorsprung und Aufnahme in formschlüssigem Eingriff miteinander gedrückt werden.

Durch diese Gestaltung ist es möglich, das Seitenwandteil mit einer Hand in die geschlossene Stellung zu schwenken, wobei sich der Verriegelungsmechanismus selbsttätig schließt, indem der Schließvorsprung mit der Aufnahme in Eingriff kommt.

Ferner führt das Seitenwandteil eine reine Schwenkbewegung aus, ohne dass am Ende bei Erreichen der geschlossenen Stellung das Seitenwandteil als Ganzes vertikal abgesenkt wird wie im gattungsbildenden Stand der Technik. Es wird nur der Schließvorsprung oder die Aufnahme angehoben oder abgesenkt. Dadurch ergeben sich weniger Erschütterungen und weniger störende Geräusche und die Bedienungskräfte sind deutlich geringer. Das wird letztlich dadurch ermöglicht, dass an die Stelle der Vertikalbewegung des Seitenwandteils als Ganzes eine Relativbewegung von Schließvorsprung und Aufnahme zueinander tritt, so dass das Seitenwandteil nur noch eine reine Schwenkbewegung ausführt.

In einer bevorzugten Ausführungsform weist der Verriegelungsmechanismus einen manuell zu betätigenden Hebel auf, der bei Betätigung auf die bewegliche Komponente von Schließvorsprung und Aufnahme einwirkt, um Schließvorsprung und Aufnahme relativ zueinander zu bewegen und diese dadurch aus ihrem Eingriff zu lösen; so dass das Seitenwandteil nach unten geschwenkt werden kann. Dadurch kann die Seitenwand auch geöffnet werden, ohne sie komplett anzuheben.

Der Schließvorsprung kann an dem Seitenwandteil und die Aufnahme an einer feststehenden Struktur des Wärmetherapiegeräts aufgehängt sein oder umgekehrt. Ferner kann der Schließvorsprung beweglich aufgehängt sein, während die Aufnahme ortsfest angebracht ist, oder umgekehrt. Diese verschiedenen Ausgestaltungsmöglichkeiten sind in den Ansprüche 3 bis 14 aufgeführt. Grundsätzlich können auch sowohl der Schließvorsprung als auch die Aufnahme geradlinig beweglich aufgehängt sein, solange die Führungskurve dafür sorgt, dass bei der Bewegung des Seitenwandteils in die geschlossene Stellung der Schließvorsprung vor der offenen Seite der Aufnahme steht, so dass die Vorspanneinrichtung die beiden Komponenten dann in Eingriff miteinander drücken kann.

Die Erfindung wird im Folgenden anhand eines Ausführungsbeispiels in den Zeichnungen erläutert, in denen:
Fig. 1 eine schematische Seitenansicht eines Wärmetherapiegerätes zeigt,
Fig. 2 schematische Ansichten eines Verriegelungsmechanismus für ein Wärmetherapiegerät gemäß der Erfindung in Draufsicht und von der Seite zeigt,
Fig. 3 schematische Ansichten für einen bevorzugten Verriegelungsmechanismus für ein Wärmetherapiegerät gemäß der Erfindung in Draufsicht und von der Seite zeigt,
Fig. 4 eine Detailansicht eines Wärmetherapiegerätes mit einem Verriegelungsmechanismus für ein Seitenwandteil gemäß dem Stand der Technik zeigt.

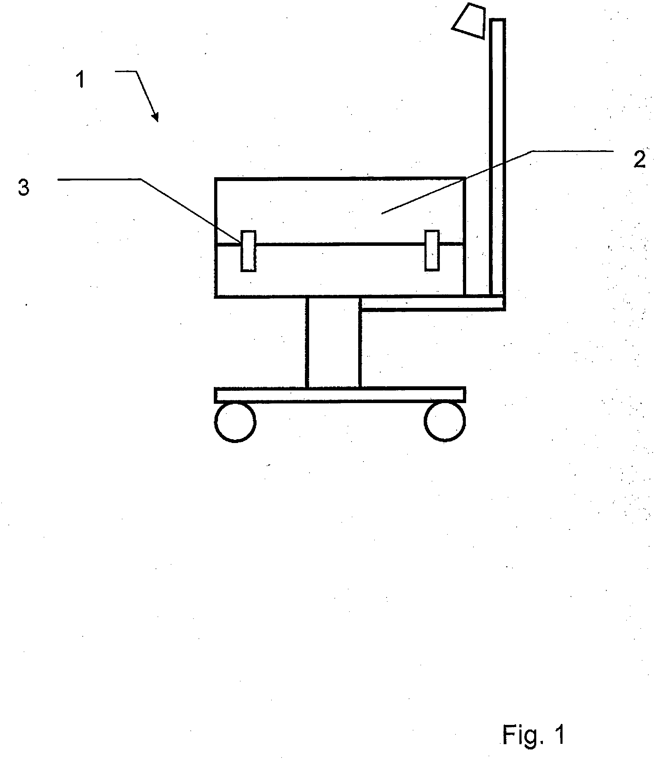

Fig. 1 zeigt eine schematische Seitenansicht eines Wärmetherapiegerätes 1. Das Wärmetherapiegerät 1 weist eine Therapieeinheit auf einem Fahrgestell und eine an einer Trägersäule aufgehängte Strahlungsheizung auf. Die Therapieeinheit umfasst eine von Seitenwänden umrandete Liegefläche. Ein Seitenwandteil 2 ist mit Scharnieren 3 schwenkbar aufgehängt, so dass es aus einer aufrechtstehenden geschlossenen Stellung in eine nach unten weggeklappte Stellung schwenkbar ist.

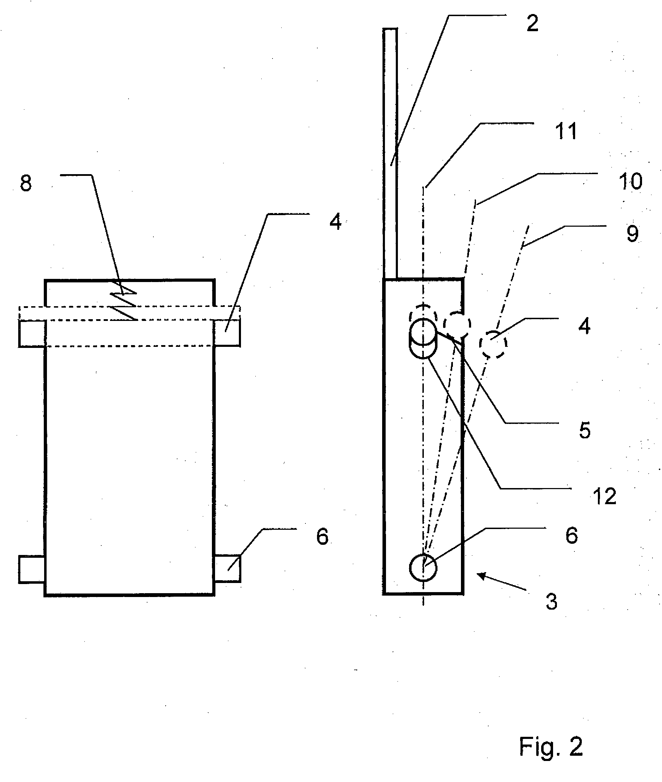

Fig. 2 zeigt ein Seitenwandteil 2 mit Scharnier 3 und einem Verriegelungsmechanismus in Ansicht von vorne und in Seitenansicht. Das Scharnier 3 ist an einer Schwenkachse 6, die fest mit dem Wärmetherapiegerät verbunden ist, aufgehängt. Der Verriegelungsmechanismus umfasst einen mit dem Scharnier verbundenen Schließvorsprung 4 in Form eines Zapfens, wobei der Zapfen 4 in Richtung der Verbindungsgeraden zwischen dem Zapfen 4 und der Schwenkachse 6 geradlinig beweglich aufgehängt ist. Ferner ist eine ortfest mit dem Wärmetherapiegerät verbundene Aufnahme 12 in Form einer Nut vorhanden. An die Nut schließt sich eine Führungskurve 5 an, die in Bezug auf die Aufnahme 12 feststehend ist. Wenn das Seitenwandteil 2 aus seiner offenen, weggeschwenkten Stellung wieder in die geschlossene Stellung gebracht wird, durchläuft das Scharnier 3 bei Annäherung an die geschlossene Stellung die mit den gestrichelten Linien 9, 10 und 11 angedeuteten Stellungen. In der Stellung 9 bewegt sich der Zapfen 4 noch frei und wird mit dem Scharnier 3 geschwenkt. Bei Auftreffen des Zapfens 4 auf die Führungskurve 5 wird der Zapfen 4 nun entgegen der Vorspannung einer Vorspannungseinrichtung 8 in größere Entfernung zu der Schwenkachse 6, im Wesentlichen nach oben gedrückt. Wenn das Scharnier 3 die mit der gestrichelten Linie 11 angedeutete aufrechte geschlossene Stellung erreicht hat ist der Zapfen 4 mit der offenen Seite der Aufnahme 12 ausgerichtet. Bei Erreichen dieser Stellung wird der Zapfen 4 dann durch die Vorspannungseinrichtung 8 in die Aufnahme 12 gedrückt, da er die Führungskurve 5 verlassen hat.

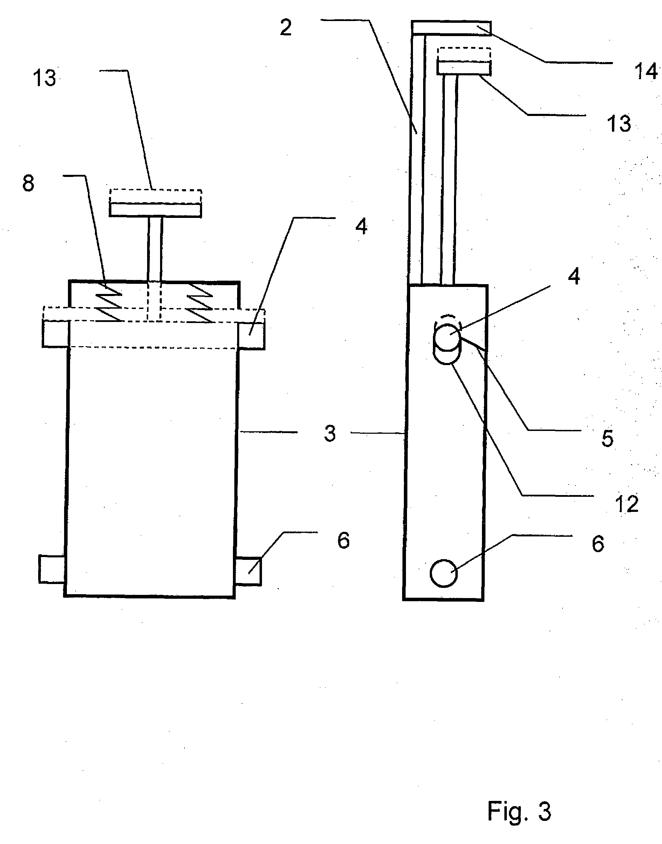

Zum Öffnen des Seitenwandteils 2 aus Figur 2 muss der Zapfen 4 aus der Aufnahme 12 gelöst werden. Dazu kann der der Zapfen manuell entgegen der Vorspannung angehoben werden, beispielsweise indem auf einen überstehenden Bereich des Zapfens manuell eine nach oben gerichtete Kraft ausgeübt wird. Grundsätzlich wäre es auch möglich, anders als in Figur 2 gezeigt, das Scharnier mit einem Langloch an der Schwenkachse 6 zu lagern, so dass das Scharnier insgesamt angehoben werden kann, um den Zapfen aus der Aufnahme 12 anzuheben. Bevorzugt ist jedoch eine rein schwenkbare Lagerung des Scharniers 3, da dann auch beim Öffnen des Seitenwandteils ein Anheben des Seitenwandteils 2 vermieden wird. Das zum Öffnen des Seitenwandteils 2 dann notwendige Anhaben des Zapfens 4 aus der Aufnahme 12 kann insbesondere in der weitergebildeten Ausführungsform nach Figur 3 einfach ausgeführt werden.

In Fig. 3 ist eine Weiterbildung eines Seitenwandteils 2 für ein erfindungsgemäßes Wärmetherapiegerät mit einer Draufsicht auf das Scharnier und einer Seitenansicht auf Scharnier und Verriegelungsmechanismus gezeigt. Das Scharnier 3 ist in der Seitenansicht in der verriegelten Stellung mit dem Zapfen 4 in Eingriff mit der Aufnahme 12 gezeigt. Das Scharnier 3 ist hier mit einem in vertikaler Richtung verschiebbaren Hebel 13 versehen, der aus der in Fig. 3 dargestellten Stellung in eine mit gestrichelten Linien angedeutete angehobene Stellung verschiebbar ist. Der Hebel 13 ist so mit dem Zapfen 4 verbunden, dass bei Anheben des Hebels 13 der Zapfen 4 ebenfalls angehoben wird, was durch die in Strichlinien angedeutete Kontur des Zapfens dargestellt ist. In der angehobenen Stellung des Zapfens 4 ist dieser soweit aus der Aufnahme 12 angehoben, dass er über die der Aufnahme 12 zugewandte Kante der Führungskurve 5 bewegt werden kann. Der Verriegelungsmechanismus kann also geöffnet werden, indem der Benutzer den Hebel 13 in Richtung zu einem Gegenstück 14 zieht, z.B. indem der Daumen auf dem Gegenstück 14 aufliegt und der Hebel mit den anderen Fingern hochgezogen wird. Nach dem Anziehen des Hebels 13 kann das Seitenwandteil 2 mit dem Scharnier 3 um die Schwenkachse 6 nach unten geschwenkt werden. Beim Zurückschwenken in die geschlossene Stellung gleitet der Zapfen 4 bei Erreichen des äußeren Endes der Führungskurve 5 auf diese auf und wird dann beim Weiterschwenken über die Führungskurve von dieser entgegen der Vorspannungseinrichtung 8 angehoben, so dass er bei Erreichen der Endstellung die äußere Kante der Führungskurve 5 oberhalb der offenen Seiten der Aufnahme 12 verlässt, wonach der bewegliche Zapfen in die Aufnahme 12 gedrückt wird und dadurch eine Verriegelung des Scharniers 3 bewirkt.

Grundsätzlich wäre es auch möglich, ein Seitenwandteil 2 durch einen Magnetverschluss in der geschlossenen Stellung zu halten. Dazu wäre ein Magnet an dem schwenkbaren Scharnier und ein Magnet an einer ortsfesten Struktur des Wärmetherapiegerätes so angebracht, dass die Magnete in der geschlossenen Stellung des Seitenwandteils mit entgegengesetzter Polausrichtung zueinander ausgerichtet sind, so dass das Scharnier durch die einander zugewandten Magnete in der geschlossenen Stellung gehalten wird. Nach Aufbringen einer Mindestkraft, die durch die Stärke und die Anordnung der Magnete zueinander bestimmte wird, kann das Scharnier mit dem Seitenwandteil daran aus der geschlossenen Stellung in die offene weggeklappte Stellung geschwenkt werden.

### Bezugszeichenliste

- 1: Wärmetherapiegerät
- 2: Seitenwandteil
- 3: Scharnier
- 4: Schließvorsprung
- 5: Führungskurve
- 6: Schwenkachse des Scharniers
- 8: Vorspanneinrichtung
- 9, 10: Zwischenstellungen des Seitenwandteils
- 11: Geschlossene Stellung des Seitenwandteils
- 12: Aufnahme
- 13: Hebel
- 14: Gegenstück
- 16: Liegefläche

## Patentansprüche

1. Wärmetherapiegerät, insbesondere Inkubator, Wärmebettchen oder eine offene Pflegeeinheit, zur Behandlung von Neugeborenen mit einer von Seitenwänden umrandeten Liegefläche zur Aufnahme eines Neugeborenen, wobei wenigstens ein Seitenwandteil (2) mit wenigstens einem zugehörigen Scharnier (3) nach unten schwenkbar aufgehängt ist und ein lösbarer Verriegelungsmechanismus zum Verriegeln des Seitenwandteils in der aufrechten geschlossenen Stellung vorhanden ist, der einen Schließvorsprung (4), eine komplementär geformte Aufnahme (12), die an einer Seite offen ist, und eine Führungskurve (5) aufweist, die dazu ausgestaltet sind, dass der Schließvorsprung (4) bei Erreichen der geschlossenen Stellung des Seitenwandteils durch die offene Seite der Aufnahme zum formschlüssigen Eingriff in die Aufnahme (12) eintreten kann, um so das Seitenwandteil in der aufrechten geschlossenen Stellung an einer feststehenden Struktur des Wärmetherapiegeräts zu verriegeln, **dadurch gekennzeichnet, dass** der Schließvorsprung (4) oder die Aufnahme (12) geradlinig beweglich in Richtung ihrer Verbindungsgeraden zur Schwenkachse (6) des Scharniers (3) aufgehängt ist, so dass der Schließvorsprung (4) in eine Stellung vor der offenen Seite der Aufnahme beweglich ist, und mit einer Vorspannungseinrichtung (8) versehen ist, die dafür sorgt, dass, wenn Schließvorsprung (4) und die offene Seite der Aufnahme (12) zueinander ausgerichtet sind, Schließvorsprung und Aufnahme in formschlüssigen Eingriff miteinander gedrückt werden.

2. Wärmetherapiegerät nach Anspruch 1, **dadurch gekennzeichnet, dass** der Verriegelungsmechanismus einen manuell zu betätigenden Hebel (13) aufweist, der bei Betätigung auf die bewegliche Komponente von Schließvorsprung (4) und Aufnahme (12) einwirkt, um den formschlüssigen Eingriff von Schließvorsprung und Aufnahme zu lösen, so dass das Seitenwandteil nach unten geschwenkt werden kann.

3. Wärmetherapiegerät nach einem der vorhergehenden Ansprüche, **dadurch gekennzeichnet, dass** der Verriegelungsmechanismus so ausgestaltet ist, dass der Schließvorsprung (4) geradlinig beweglich am Seitenwandteil (2) aufgehängt ist und die Aufnahme (12) ortsfest an einer feststehenden Struktur des Wärmetherapiegerätes angebracht ist.

4. Wärmetherapiegerät nach Anspruch 3, **dadurch gekennzeichnet, dass** der Verriegelungsmechanismus so ausgestaltet ist, dass die Aufnahme (12) nach oben offen orientiert ist, die Führungskurve (5) den Schließvorsprung (4) vor Erreichen der Aufnahme entgegen der Wirkung der Vorspannungseinrichtung (8) nach oben drückt und die Vorspannungseinrichtung den Schließvorsprung (4) bei Erreichen der nach oben offenen Aufnahme (12) nach unten in die Aufnahme drückt.

5. Wärmetherapiegerät nach Anspruch 3, **dadurch gekennzeichnet, dass** der Verriegelungsmechanismus so ausgestaltet ist, dass die Aufnahme nach unten offen orientiert ist, die Führungskurve den Schließvorsprung vor Erreichen der Aufnahme entgegen der Wirkung der Vorspannungseinrichtung nach unten drückt und die Vorspannungseinrichtung den Schließvorsprung bei Erreichen der Aufnahme nach oben in die Aufnahme drückt.

6. Wärmetherapiegerät nach Anspruch 1 oder 2, **dadurch gekennzeichnet, dass** der Verriegelungsmechanismus so ausgestaltet ist, dass der Schließvorsprung fest am Seitenwandteil und die Aufnahme geradlinig beweglich an einer feststehenden Struktur des Wärmetherapiegerätes angebracht sind.

7. Wärmetherapiegerät nach Anspruch 6, **dadurch gekennzeichnet, dass** der Verriegelungsmechanismus so ausgestaltet ist, dass die Aufnahme nach oben offen orientiert ist, die Führungskurve die Aufnahme, bevor der Schließvorsprung diese erreicht, entgegen der Wirkung der Vorspannungseinrichtung nach unten drückt und die Vorspannungseinrichtung die Aufnahme, wenn der Schließvorsprung diese erreicht, nach oben in Eingriff mit dem Schließvorsprung drückt.

8. Wärmetherapiegerät nach Anspruch 6, **dadurch gekennzeichnet, dass** der Verriegelungsmechanismus so ausgestattet ist, dass die Aufnahme nach unten offen orientiert ist, die Führungskurve die Aufnahme, bevor der Schließvorsprung diese erreicht, entgegen der Wirkung der Vorspannungseinrichtung nach oben drückt und die Vorspannungseinrichtung die Aufnahme, wenn der Schließvorsprung diese erreicht, nach unten gegen den Schließvorsprung drückt.

9. Wärmetherapiegerät nach Anspruch 1 oder 2, **dadurch gekennzeichnet, dass** die Aufnahme geradlinig beweglich am Seitenwandteil aufgehängt ist und der Schließvorsprung fest an einer feststehenden Struktur des Wärmetherapiegeräts angebracht ist.

10. Wärmetherapiegerät nach Anspruch 9, **dadurch gekennzeichnet, dass** der Verriegelungsmechanismus so ausgestaltet ist, dass die Aufnahme nach oben offen orientiert ist, die Führungskurve die Aufnahme vor Erreichen des Schließvorsprungs entgegen der Wirkung der Vorspannungseinrichtung nach unten drückt und die Vorspannungseinrichtung die Aufnahme bei Erreichen des Schließvorsprungs nach oben gegen den Schließvorsprung drückt.

11. Wärmetherapiegerät nach Anspruch 9, **dadurch gekennzeichnet, dass** Aufnahme nach unten offen orientiert ist, die Führungskurve die Aufnahme vor Erreichen des Schließvorsprungs entgegen der Wirkung der Vorspannungseinrichtung nach oben drückt und die Vorspannungseinrichtung die Aufnahme bei Erreichen des Schließvorsprungs nach unten gegen den Schließvorsprung drückt.

12. Wärmetherapiegerät nach Anspruch 1 oder 2, **dadurch gekennzeichnet, dass** die Aufnahme fest an dem Seitenwandteil und der Schließvorsprung geradlinig beweglich an einer feststehenden Struktur des Wärmetherapiegeräts angebracht ist.

13. Wärmetherapiegerät nach Anspruch 12, **dadurch gekennzeichnet, dass** der Verriegelungsmechanismus so ausgestaltet ist, dass die Aufnahme nach oben offen orientiert ist, die Führungskurve den Schließvorsprung, bevor die Aufnahme diesen erreicht, entgegen der Wirkung der Vorspannungseinrichtung nach oben drückt und die Vorspannungseinrichtung den Schließvorsprung, wenn die Aufnahme diesen erreicht, nach unten in die Aufnahme drückt.

14. Wärmetherapiegerät nach Anspruch 12, **dadurch gekennzeichnet, dass** der Verriegelungsmechanismus so ausgestaltet ist, dass die Aufnahme nach unten offen orientiert ist, die Führungskurve den Schließvorsprung, bevor die Aufnahme diesen erreicht, entgegen der Wirkung der Vorspannungseinrichtung nach unten drückt und die Vorspannungseinrichtung den Schließvorsprung, wenn die Aufnahme diesen erreicht, nach oben in die Aufnahme drückt.

## Claims

1. Thermotherapy device, especially incubator, heating bed or an open care unit, for the treatment of newborn babies, with a reclining surface bordered by side walls for receiving a newborn baby, wherein at least one side wall part (2) is suspended with at least one associated hinge (3) such that it can be pivoted downward and a releasable locking mechanism for locking the side wall part is present in the upright closed position, which said locking mechanism has a closing projection (3), a complementarily shaped receptacle (12), which is open on one side, and a guide curve 5, which is designed such that when the closed position of the side wall part is reached it can enter the receptacle (12) through the open side of the receptacle for positive-locking meshing in order to thus lock the side wall part in the upright closed position on a stationary structure of the thermotherapy device, the closing projection (4) or receptacle (12) is suspended linearly movably in the direction of its connection lines with the pivot axis (6) of the hinge (3), so that the closing projection (4) can be moved into a position in front of the open side of the receptacle, and is provided with a prestressing device (8), which ensures that when the closing projection (4) and the open side of the receptacle (12) are oriented towards one another, the closing projection and the receptacle are pressed together into a positive-locking meshing with one another.

2. Thermotherapy device in accordance with claim 1, **characterized in that** the locking mechanism has a lever (13), which can be actuated manually and which acts on the movable component of the closing projection (4) and receptacle (12) during actuation in order to release the positive-locking meshing of the closing projection and receptacle, so that the side wall part can be pivoted downward.

3. Thermotherapy device in accordance with one of the above claims, **characterized in that** the locking mechanism is designed such that the closing projection (4) is suspended linearly movably on the side wall part (2) and the receptacle is arranged stationarily on a stationary structure of the thermotherapy device.

4. Thermotherapy device in accordance with claim 3, **characterized in that** the locking mechanism is designed such that the receptacle (12) is oriented such that it is upwardly open, the guide curve (5) presses the closing projection (4) upwardly against the action of the prestressing device (8) before the receptacle is reached, and the prestressing device presses the closing projection (4) downwardly into the receptacle when the upwardly open receptacle (12) is reached.

5. Thermotherapy device in accordance with claim 3, **characterized in that** the locking mechanism is designed such that the receptacle is oriented such that it is downwardly open, the guide curve presses the closing projection downward against the action of the prestressing device before the receptacle is reached, and the prestressing device presses the closing projection upward into the receptacle when the receptacle is reached.

6. Thermotherapy device in accordance with claim 1 or 2, **characterized in that** the locking mechanism is designed such that the closing projection is arranged rigidly on the side wall part and the receptacle is arranged linearly movably on a stationary structure of the thermotherapy device.

7. Thermotherapy device in accordance with claim 6, **characterized in that** the locking mechanism is designed such that the receptacle is oriented such that it is upwardly open, the guide curve presses the receptacle downward against the action of the prestressing device before the closing projection reaches this [guide curve], and the prestressing device presses the receptacle, when the closing projection reaches it, upward to mesh with the closing projection.

8. Thermotherapy device in accordance with claim 6, **characterized in that** the locking mechanism is designed such that the receptacle is oriented such that it is downwardly open, the guide curve presses the receptacle, before the closing projection reaches it, upwardly against the action of the prestressing device, and the prestressing device presses the receptacle, before the closing projection reaches it, downwardly against the closing projection.

9. Thermotherapy device in accordance with claim 1 or 2, **characterized in that** the receptacle is suspended linearly movably on the side wall part and the closing projection is arranged rigidly on a stationary structure of the thermotherapy device.

10. Thermotherapy device in accordance with claim 9, **characterized in that** the locking mechanism is designed such that the receptacle is oriented such that it is upwardly open, the guide curve presses the receptacle against the action of the prestressing device before the closing projection is reached, and the prestressing device presses the receptacle upwardly against the closing projection when the closing projection is reached.

11. Thermotherapy device in accordance with claim 9, **characterized in that** the receptacle is oriented such that it is downwardly open, the guide curve presses the receptacle upwardly against the action of the prestressing device before the closing projection is reached, and the prestressing device presses the receptacle downwardly against the closing projection when the closing projection is reached.

12. Thermotherapy device in accordance with claim 1 or 2, **characterized in that** the receptacle is arranged rigidly on the side wall part and the closing projection is arranged linearly movably on a stationary structure of the thermotherapy device.

13. Thermotherapy device in accordance with claim 12, **characterized in that** the locking mechanism is designed such that the receptacle is oriented such that it is upwardly open, the guide curve presses the closing projection, before the receptacle reaches it, upwardly against the action of the prestressing device, and the prestressing device presses the closing projection, when the receptacle reaches it, downwardly into the receptacle.

14. Thermotherapy device in accordance with claim 12, **characterized in that** the locking mechanism is designed such that the receptacle is oriented such that it is downwardly open, the guide curve presses the closing projection, before the receptacle reaches it, downwardly against the action of the prestressing device, and the prestressing device presses the closing projection, when the receptacle reaches it, upwardly into the receptacle.

## Revendications

1. Appareil de thermothérapie, notamment incubateur, couveuse ou une unité de soins ouverte, destiné au traitement des nouveau-nés et comprenant une surface de couchage entourée de parois latérales destinée à accueillir un nouveau-né, au moins une partie de paroi latérale (2) étant accrochée avec au moins une charnière (3) associée de manière à pouvoir pivoter vers le bas et un mécanisme de verrouillage détachable étant présent pour verrouiller la partie de paroi latérale dans la position fermée relevée, lequel possède un élément en saillie de fermeture (4), un logement (12) de forme complémentaire qui est ouvert d'un côté et une courbe de guidage (5), lesquels sont configurés de telle sorte que l'élément en saillie de fermeture (4), lorsque la position de fermeture de la partie de paroi latérale est atteinte, peut pénétrer à travers le côté ouvert du logement pour venir en prise par complémentarité de formes dans le logement (12) afin de verrouiller ainsi la partie de paroi latérale dans la position fermée relevée contre une structure fixe de l'appareil de thermothérapie, **caractérisé en ce que** l'élément en saillie de fermeture (4) ou le logement (12) sont accrochés avec mobilité rectiligne dans la direction de leurs droites de liaison par rapport à l'axe de pivotement (6) de la charnière (3), de sorte que l'élément en saillie de fermeture (4) soit mobile dans une position devant le côté ouvert du logement et pourvu d'un dispositif de précontrainte (8) qui contribue à ce que l'élément en saillie de fermeture et le logement soient poussés l'un contre l'autre dans une prise par complémentarité de formes lorsque l'élément en saillie de fermeture (4) et le côté ouvert du logement (12) sont orientés l'un vers l'autre.

2. Appareil de thermothérapie selon la revendication 1, **caractérisé en ce que** le mécanisme de verrouillage possède un levier (13) à actionner manuellement qui, lorsqu'il est actionné, agit sur les composants mobiles de l'élément en saillie de fermeture (4) et du logement (12) afin de libérer la prise par complémentarité de formes de l'élément en saillie de fermeture et du logement, de sorte que la partie de paroi latérale puisse être pivotée vers le bas.

3. Appareil de thermothérapie selon l'une des revendications précédentes, **caractérisé en ce que** le mécanisme de verrouillage est configuré de telle sorte que l'élément en saillie de fermeture (4) est accroché ou suspendu avec mobilité rectiligne à la partie de paroi latérale (2) et le logement (12) est monté en position fixe sur une structure fixe de l'appareil de thermothérapie.

4. Appareil de thermothérapie selon la revendication 3, **caractérisé en ce que** le mécanisme de verrouillage est configuré de telle sorte que le logement (12) est orienté en étant ouvert vers le haut, la courbe de guidage (5) pousse l'élément en saillie de fermeture (4) vers le haut contre l'effet du dispositif de précontrainte (8) avant d'atteindre le logement, et le dispositif de précontrainte pousse l'élément en saillie de fermeture (4) vers le bas dans le logement au moment d'atteindre le logement (12) ouvert vers le haut.

5. Appareil de thermothérapie selon la revendication 3, **caractérisé en ce que** le mécanisme de verrouillage est configuré de telle sorte que le logement est orienté en étant ouvert vers le bas, la courbe de guidage pousse l'élément en saillie de fermeture vers le bas contre l'effet du dispositif de précontrainte avant d'atteindre le logement, et le dispositif de précontrainte pousse l'élément en saillie de fermeture vers le haut dans le logement au moment d'atteindre le logement.

6. Appareil de thermothérapie selon la revendication 1 ou 2, **caractérisé en ce que** le mécanisme de verrouillage est configuré de telle sorte que l'élément en saillie de fermeture est monté à demeure sur la partie de paroi latérale et le logement est monté avec mobilité rectiligne sur une structure fixe de l'appareil de thermothérapie.

7. Appareil de thermothérapie selon la revendication 6, **caractérisé en ce que** le mécanisme de verrouillage est configuré de telle sorte que le logement est orienté en étant ouvert vers le haut, la courbe de guidage pousse le logement vers le bas contre l'effet du dispositif de précontrainte avant que l'élément en saillie de fermeture l'atteigne, et le dispositif de précontrainte pousse le logement vers le haut en prise avec l'élément en saillie de fermeture lorsque l'élément en saillie de fermeture l'atteint.

8. Appareil de thermothérapie selon la revendication 6, **caractérisé en ce que** le mécanisme de verrouillage est configuré de telle sorte que le logement est orienté en étant ouvert vers le bas, la courbe de guidage pousse le logement vers le haut contre l'effet du dispositif de précontrainte avant que l'élément en saillie de fermeture l'atteigne, et le dispositif de précontrainte pousse le logement vers le bas contre l'élément en saillie de fermeture lorsque l'élément en saillie de fermeture l'atteint.

9. Appareil de thermothérapie selon la revendication 1 ou 2, **caractérisé en ce que** le logement est accroché avec mobilité rectiligne à la partie de paroi latérale et l'élément en saillie de fermeture est monté à demeure sur une structure fixe de l'appareil de thermothérapie.

10. Appareil de thermothérapie selon la revendication 9, **caractérisé en ce que** le mécanisme de verrouillage est configuré de telle sorte que le logement est orienté en étant ouvert vers le haut, la courbe de guidage pousse le logement vers le bas contre l'effet du dispositif de précontrainte avant que l'élément en saillie de fermeture l'atteigne, et le dispositif de précontrainte pousse le logement vers le haut contre l'élément en saillie de fermeture lorsque l'élément en saillie de fermeture l'atteint.

11. Appareil de thermothérapie selon la revendication 9, **caractérisé en ce que** le logement est orienté en étant ouvert vers le bas, la courbe de guidage pousse le logement vers le haut contre l'effet du dispositif de précontrainte avant que l'élément en saillie de fermeture l'atteigne, et le dispositif de précontrainte pousse le logement vers le bas contre l'élément en saillie de fermeture lorsque l'élément en saillie de fermeture l'atteint.

12. Appareil de thermothérapie selon la revendication 1 ou 2, **caractérisé en ce que** le logement est accroché à demeure à la partie de paroi latérale et l'élément en saillie de fermeture est monté avec mobilité rectiligne sur une structure fixe de l'appareil de thermothérapie.

13. Appareil de thermothérapie selon la revendication 12, **caractérisé en ce que** le mécanisme de verrouillage est configuré de telle sorte que le logement est orienté en étant ouvert vers le haut, la courbe de guidage pousse l'élément en saillie de fermeture vers le haut contre l'effet du dispositif de précontrainte avant que le logement l'atteigne, et le dispositif de précontrainte pousse l'élément en saillie de fermeture vers le bas dans le logement lorsque le logement l'atteint.

14. Appareil de thermothérapie selon la revendication 12, **caractérisé en ce que** le mécanisme de verrouillage est configuré de telle sorte que le logement est orienté en étant ouvert vers le bas, la courbe de guidage pousse l'élément en saillie de fermeture vers le bas contre l'effet du dispositif de précontrainte avant que le logement l'atteigne, et le dispositif de précontrainte pousse l'élément en saillie de fermeture vers le haut dans le logement lorsque le logement l'atteint.
